# EUROPÄISCHE PATENTANMELDUNG

(11) **EP 2 954 927 A1**
(43) Veröffentlichungstag der Anmeldung: **16.12.2015**
(21) Anmeldenummer: 15171698.2
(22) Anmeldetag: 11.06.2015
(51) Int. Cl.: A61M 37/00

(54) **ANTRIEBSMODUL FÜR EIN HANDGERÄT ZUM WIEDERHOLTEN AUFSTECHEN EINER MENSCHLICHEN ODER TIERISCHEN HAUT, HANDGERÄT UND VERFAHREN**

(30) Priorität: 12.06.2014 EP 14172106
(71) Anmelder: MT.DERM GmbH, 14167 Berlin (DE)
(72) Erfinder: SCHERKOWSKI, Dirk, 12489 Berlin (DE); KNOTHE, Kornelius, 12167 Berlin (DE)
(74) Vertreter: Bittner, Thomas L.

(57) **Zusammenfassung**

Die Anmeldung betrifft ein Antriebsmodul für ein Handgerät zum wiederholten lokalen Aufstechen einer menschlichen oder tierischen Haut mit einem Modulgehäuse, einer Antriebseinrichtung, einem Wandlungsmechanismus, und einer Kopplungseinrichtung, wobei der Wandlungsmechanismus einen mit der Antriebswelle (21) funktionell verbundenes und zur axialen Richtung der Antriebswelle (21) schräg gestellten Kurbelzapfen (24) aufweist, welcher infolge der rotierenden Antriebsbewegung in eine Bewegung um eine zur axialen Richtung der Antriebswelle (21) parallele Achse versetzt wird, und wobei der Kurbelzapfen (24) an ein quer zur axialen Richtung der Antriebswelle (21) hin und her verlagerbares und an ein mit der Nadelaufnahme des Stechmoduls koppelbares Kopplungsbauteil (25) koppelt, derart, dass im Betrieb die Bewegung des Kurbelzapfens (24) im Bereich eines Abgriffs in eine Vor- und Zurückbewegung des Kopplungsbauteils (25) quer zur axialen Richtung der Antriebswelle (21) gewandelt wird. Es ist eine Hubeinstellvorrichtung vorgesehen, mit der ein Hub der Vor- und Zurückbewegung der Nadelaufnahme quer zur axialen Richtung der Antriebswelle (21) einstellbar ist. Weiterhin betrifft die Anmeldung ein Handgerät sowie ein Verfahren zum Betreiben eines Handgeräts.

## Beschreibung

Die Erfindung betrifft ein Antriebsmodul für ein Handgerät zum wiederholten Aufstechen einer menschlichen oder tierischen Haut, ein Handgerät sowie Verfahren.

### Hintergrund

Vorrichtungen zum lokalen Aufstechen einer menschlichen oder einer tierischen Haut werden in der Regel als Handgeräte ausgeführt. Vom Bedienpersonal können derartige Handgeräte zum Aufbringen einer Farbe für eine Tätowierung (Tattoo) und / oder permanentes Make-up im Bereich der Hautoberfläche verwendet werden. Aber auch das Einbringen kosmetischer oder medizinischer Wirkstoffe über die Haut ist mit solchen Geräten möglich, indem die haut lokal aufgestochen wird. Darüber hinaus können solche Geräte verwendet werden, ohne dass irgendeine Substanz eingebracht wird, zum Beispiel zur Hautstimulierung.

Ein Handgerät zum lokalen Aufstechen einer Haut ist beispielsweise aus der Druckschrift DE 299 19 199 U1 bekannt. Das bekannte Handgerät weist ein Griffstück, eine Antriebseinrichtung und eine Stechnadel, die mit Hilfe der Antriebseinrichtung im Betrieb relativ zu einer Nadeldüse vor und zurück bewegt wird, wobei mindestens zwei lösbar miteinander verbundene Module vorgesehen sind, und das eine der beiden Module als wieder verwendbares Grundmodul mit integrierter Antriebseinrichtung ausgebildet ist. Das andere der beiden Module ist ein sterilisiertes Einwegmodul, in das bei dem bekannten Handgerät alle durch die Köperflüssigkeiten eines Kunden kontaminierbaren Bestandteile integriert sind. Auf diese Weise wird das Handgerät in Form von zwei Modulen zur Verfügung gestellt, von denen eines, nämlich das Einwegmodul, nach der Benutzung ausgetauscht werden kann, während das andere Modul, welches die Antriebseinrichtung umfasst, wieder verwendet wird. Mit Hilfe des Einwegmoduls werden die hygienischen Bedingungen beim Aufbringen einer Tätowierung und / oder von permanentem Make-up verbessert, da alle Teile ausgetauscht werden, die potentiell durch die bei der Behandlung austretende Körperflüssigkeit des Kunden kontaminiert werden können. Es wird so vermieden, dass das gesamte Handgerät ausgetauscht werden muss.

Aus dem Dokument EP 1 495 782 A1 ist ein Antriebsmodul für eine Vorrichtung zum lokalen Aufstechen einer menschlichen oder einer tierischen Haut bekannt, bei dem eine Antriebseinrichtung, mit der eine Antriebsbewegung erzeugbar ist, und ein an die Antriebseinrichtung gekoppelter Wandlungsmechanismus vorgesehen sind, mit dem die Antriebsdrehbewegung in einer auf eine die Haut lokal aufstechende Stecheinrichtung einkoppelbare Vorwärts- / Rückwärtsbewegung umgewandelt wird, so dass eine repetierende Bewegung einer Stechnadel ermöglicht ist. Der Wandlungsmechanismus umfasst ein Funktionsbauteil, welches bei der Bewegungswandlung eine Taumel- oder Kippbewegung ausführt, wodurch eine Antriebskraft zum Bewegen einer die Haut lokal aufstechenden Nadel in Vorwärts- und Rückwärtsrichtung zur Verfügung gestellt wird. Das Funktionsbauteil ist in einer Ausführung mittels eines Kugellagers freilaufend gelagert. Ein nicht beabsichtigtes Verdrehen des Funktionsbauteils, welches durch die Antriebsdrehbewegung hervorgerufen werden kann, wird bei der bekannten Vorrichtung dadurch verhindert, dass das mittels eines Kugellagers montierte Funktionsbauteil oder ein hieran gebildeter Vorsprung in eine Ausnehmung eingreifen. Es hat sich gezeigt, dass diese Art der Sicherung des Funktionsbauteils beim Betrieb der Vorrichtung zu einer nicht unerheblichen Geräuschbelastung führt, die insbesondere durch das Hin- und Herbewegen des Vorsprungs in der Ausnehmung verursacht wird.

Das Dokument CN 201 759 989 U offenbart eine Tätowiervorrichtung mit einer Antriebseinrichtung. Die Antriebseinrichtung ist mit einer exzentrischen Welle gebildet, welche eine Antriebsbewegung für eine Nadel bereitstellt.

Das Dokument US 5,279,552 offenbart eine intradermale Injektionsvorrichtung, die auf der Haut eines Patienten angeordnet ist und verwendet wird, um mittels einer Nadel Tinte, Farbstoff oder eine ähnliche Flüssigkeit unter die Haut eines Patienten zu injizieren.

Das Dokument US 2012/0279330 A1 bezieht sich auf einen Übertragungsmechanismus einer Augenbrauen-Tätowiervorrichtung. Die Vorrichtung ist mit einer exzentrischen Drehwelle gebildet, die mit einer Antriebswelle eines Motors gekoppelt ist.

Eine weitere Tätowiervorrichtung ist in dem Dokument KR 2013 /0058843 A offenbart.

### Zusammenfassung

Aufgabe der Erfindung ist es, ein Antriebsmodul für ein Handgerät zum wiederholten lokalen Aufstechen einer menschlichen oder tierischen Haut sowie ein Handgerät anzugeben, bei denen im Betrieb des Handgerätes der Bedienkomfort verbessert ist, insbesondere hinsichtlich störender Begleiterscheinungen, wie zum Beispiel Geräusche oder Vibrationen des Handgeräts.

Zur Lösung der Aufgabe ist ein Antriebsmodul für ein Handgerät zum wiederholten lokalen Aufstechen einer menschlichen oder tierischen Haut nach dem unabhängigen Anspruch 1 geschaffen. Weiter betrifft der unabhängige Anspruch 12 ein Handgerät. Weiterhin ist ein Verfahren zum Betreiben eines Handgeräts zum wiederholten lokalen Aufstechen einer menschlichen oder tierischen Haut nach dem unabhängigen Anspruch 14 geschaffen. Vorteilhafte Ausgestaltungen sind Gegenstand von abhängigen Unteransprüchen.

Ein Handgerät zum wiederholten lokalen Aufstechen einer menschlichen oder tierischen Haut weist ein Gehäuse mit einem hieran gebildeten Handgriff auf. Im Betrieb des Handgerätes, welches im Fall der Nutzung zum Ausbilden Tattoos oder permanentem Make-up auch als Tätowiervorrichtung bezeichnet werden kann, greift der Nutzer den Handgriff, um das Handgerät zu führen. Andere Anwendungen sehen das lokale Aufstechen der Haut zum Einbringen eines kosmetischen oder eines medizinischen Wirkstoffes vor. Aber auch für das lokale Aufstechen der menschlichen oder tierischen Haut ohne Einbringen irgendeiner Substanz kann das Handgerät genutzt werden, zum Beispiel zur Hautstimulierung. Im Gehäuse ist eine Antriebseinrichtung aufgenommen, mit der über eine Antriebswelle eine rotierende Antriebskraft bereitgestellt wird. Hierfür kann ein Elektromotor vorgesehen sein.

An die Antriebswelle koppelt ein Wandlungsmechanismus, der im Gehäuse angeordnet und eingerichtet ist, die rotierende Antriebsbewegung in eine nicht rotierende Antriebsbewegung zu wandeln. Die hierdurch bereitgestellte Antriebskraft kann zum Beispiel axial ausgerichtet sein, beispielsweise parallel zur Achse der Antriebswelle. Es ist weiterhin eine Stecheinrichtung vorgesehen, die im Gehäuse aufgenommen ist und eine oder mehrere Stechnadeln aufweist, die an einer Nadelaufnahme angeordnet sind. Im Fall von mehreren Nadeln können diese in Gruppen angeordnet sein, in denen die Nadeln einander nahestehen. Aber auch die Verteilung von Nadeln oder Nadelgruppen über die Fläche einer Nadelplatte kann vorgesehen sein. In diesem Fall ist die Nadelaufnahme mit einer Nadelplatte gebildet. Die Nadelaufnahme ist verbunden mit dem Wandlungsmechanismus und wird zusammen mit der einen oder den mehreren Stechnadeln im Betrieb entlang einer Bewegungsbahn wiederholt vor und zurück bewegt, beispielweise in axialer Richtung. Die Bewegungsbahn kann aus verschieden ausgebildeten Teilabschnitten bestehen.

Der Wandlungsmechanismus weist einen zur axialen Richtung der Antriebswelle schräg gestellten Kurbelzapfen auf. Der Kurbelzapfen steht mit der Antriebswelle funktionell in Verbindung, so dass er infolge der rotierenden Antriebsbewegung in eine Bewegung um die axiale Richtung der Antriebswelle versetzt wird. Der Kurbelzapfen koppelt im Bereich eines Abgriffs an ein quer zur axialen Richtung der Antriebswelle hin und her verlagerbares und mit der Nadelaufnahme in Verbindung stehendes Kopplungsbauteil, derart, dass die Bewegung des Kurbelzapfens in eine Vor- und Zurückbewegung des Kopplungsbauteils und somit der Nadelaufnahme quer zur axialen Richtung der Antriebswelle gewandelt wird. Die Vor- und Zurückbewegung des Kopplungsbauteils kann beispielsweise als eine lineare Bewegung ausgeführt werden. Im Betrieb bewegt sich hierdurch die Nadelaufnahme mit der einen oder den mehreren Stechnadeln quer zur axialen Richtung der Antriebswelle vor und zurück (repetierend), wobei Spitzen der Stechnadel wenigstens in der Endstellung der Vorwärtsbewegung außerhalb des Gehäuses angeordnet sein können.

Es ist eine Hubeinstellvorrichtung vorgesehen, mit der ein Hub der Vor- und Zurückbewegung der Nadelaufnahme quer zur axialen Richtung der Antriebswelle einstellbar ist. Der Hub ist die axial ausgerichtete Bewegung zwischen den Umkehrpunkten der linearen Vor- und Zurückbewegung der Nadelaufnahme oder des hieran koppelnden Kopplungsbauteils. Er beeinflusst, wie weit die Nadelspitze der einen oder der mehreren Stechnadeln im Betrieb vorwärts bewegt wird, wodurch in einer Ausgestaltung die Einstechtiefe im lokalen Bereich der aufzustechenden Haut beeinflusst werden kann. Mit Hilfe der Verstellbarkeit des Hubes der axial ausgerichteten Antriebsbewegung können das Antriebsmodul und das Handgerät für unterschiedliche Anwendungszwecke eingestellt werden.

Die Antriebseinrichtung, insbesondere ein Elektromotor, kann in einem zum Handgriff am Gehäuse querstehenden Gehäuseabschnitt aufgenommen sein. Die Antriebseinrichtung kann eingerichtet sein, im Betrieb eine drehende Antriebsbewegung von wenigstens etwa 1.800 min⁻¹ (30 Hz) bereitzustellen.

Das Gehäuse kann aus einem einzigen Material oder einer Kombination verschiedenen Materialien bestehen, wozu insbesondere Metall und Kunststoff gehören. Gehäuseabschnitte können lösbar montiert sein, um zum Beispiel Bereiche des Gehäuseinneren zu Austausch- oder Wartungszwecken freizugeben. Aus dem Gehäuse kann eine Kabelverbindung herausgeführt sein, die zum Anschluss des Handgerätes oder des Antriebsmoduls an ein externes Steuergerät dient. Die Kabelverbindung kann einerseits dem Anschließen eines Elektromotors an eine Energiequelle dienen. Andererseits kann die Kabelverbindung Datenkabel aufweisen, über die zwischen dem Handgerät oder dem Antriebsmodul und dem Steuergerät elektronische Daten austauschbar sind. Ein solcher Datenaustausch kann alternativ oder ergänzend auch über eine drahtlose Datenverbindung ausgeführt werden, zum Beispiel unter Verwendung der Bluetooth-Technologie. Steuergeräte für Handgeräte zum wiederholten lokalen Aufstechen einer menschlichen oder tierischen Haut oder Antriebsmodule sind als solche in verschiedenen Ausführungsformen bekannt und werden hier daher nicht näher erläutert.

In den verschiedenen Ausführungsformen kann die Kopplung zwischen Wandlungsmechanismus und Nadelaufnahme, die mit einem Nadelschaft gebildet sein kann, derart ausgeführt sein, dass die Nadelaufnahme aufgrund der axial ausgerichteten Antriebskraft nicht nur vorgefahren wird, sondern auch mittels des das Kopplungsbauteil umfassenden Wandlungsmechanismus zurückgezogen wird. Bei dieser Ausführungsform stellt der Wandlungsmechanismus selbst eine Rückstellkraft bereit. Alternativ oder ergänzend kann vorgesehen sein, dass die Rückstellkraft wenigstens teilweise von einem elastischen Element bereitgestellt wird, beispielsweise einer Feder oder einer Membran. Bei dieser Ausführung erfolgt das Aus- oder Vorfahren der Nadelaufnahme mit der einen oder den mehreren Nadeln gegen eine elastische Vorspannung, die ihrerseits dann zum Zurückbewegen beiträgt oder dieses allein bewirkt. Das elastische Element zieht sich nach dem Strecken beim Ausfahren der Stechnadeln selbsttätig wieder zusammen und bewirkt so die Rückbewegung der einen oder der mehreren Nadeln. Der Kurbelzapfen kann aus einem gehärteten Material bestehen, zum Beispiel einem gehärteten Stahl.

Der Kurbelzapfen kann an einem Mitnehmerbauteil aufgenommen sein, welches an die Antriebswelle koppelt. Das Mitnehmerbauteil kann als zylindrisches Bauteil ausgeführt sein, welches lösbar oder nicht lösbar auf der Antriebswelle montiert ist. Der Kurbelzapfen kann geneigt sein zu einer Oberfläche des Mitnehmerbauteils, auf welcher der Kurbelzapfen angeordnet ist. Die Oberfläche des Mitnehmerbauteils kann im Winkel von etwa 90° zur Antriebsachse angeordnet sein. Das Mintnehmerbauteil kann als Vollmaterial- oder Hohlkörper-Bauteil ausgeführt sein. Auf einer von der Antriebseinrichtung abgewandten Stirnseite kann der Kurbelzapfen, zum Beispiel in der Ausführung als Stift, in einer zugeordneten Ausnehmung aufgenommen sein, sei es lösbar oder nicht lösbar. Das Mitnehmerbauteil kann im Gehäuse des Handgerätes von einem zugeordneten Innengehäuse wenigstens teilweise umgeben sein. Das Innengehäuse kann in einer Ausführung lösbar mit der Antriebseinrichtung verbunden sein, beispielsweise mittels einer Schraubverbindung. Das Innengehäuse kann eine Schutzhülle für das im Betrieb rotierende Mitnehmerbauteil mitbilden.

Der Kurbelzapfen kann, insbesondere im Bereich des Abgriffs, über ein Kugelgelenk an das Kopplungsbauteil koppeln. Das Kugelgelenk lässt einerseits die umlaufende Bewegung des Kurbelzapfens im Betrieb zu. Andererseits bewirkt es, dass aufgrund der Bewegung das Kopplungsbauteil in linearer Richtung vor- und zurückbewegt wird.

Der Kurbelzapfen kann in einem Durchbruch an dem Kopplungsbauteil freilaufend lagern, insbesondere im Bereich des Abgriffs. Der Durchbruch an dem Kopplungsbauteil, in welches der Kurbelzapfen eingreift, kann mit einer Schlitzführung gebildet sein, in welche der Kurbelzapfen frei läuft im Betrieb und die vorzugsweise quer zur axialen Richtung der Antriebswelle und zur Bewegungsrichtung des Kopplungsbauteils ausgebildet ist.

Das Kopplungsbauteil kann in einer quer zur axialen Richtung der Antriebswelle ausgerichteten Führung angeordnet sein. Das Kopplungsbauteil kann abschnittsweise oder in seiner Gesamtheit in der Führung aufgenommen sein. Die Überlappung zwischen Kopplungsbauteil und zugeordneter Führung kann von der Betriebsstellung des Kurbelzapfens und des Kopplungsbauteils abhängen, wobei das Kopplungsbauteil in einer ersten Betriebsstellung vollständig und in einer zweiten Betriebsstellung nur teilweise innerhalb der Führung angeordnet ist. Die Führung für das Kopplungsbauteil kann als Schlitzführung ausgeführt sein.

Mittels der Hubeinstellvorrichtung kann der Abgriff entlang des Kurbelzapfens verlagerbar sein. Allgemein kann entlang des schräggestellten Kurbelzapfens die Lage oder der Ort der Kopplung (Abgriff) zwischen dem Kurbelzapfen und dem mit der Nadelaufnahme in Verbindung stehenden Kopplungsbauteil verändert. Es ändert sich hierdurch in Richtung quer zur axialen Richtung der Antriebswelle der Abstand der der Kopplung zur Antriebswelle ändert. Der Ort der Kopplung wird zur Antriebswelle hin oder von dieser weg verlagert, wodurch der ausgeführte Hub eingestellt wird.

Der Hub kann mit Hilfe der Hubeinstellvorrichtung stufenlos einstellbar sein.

Es kann vorgesehen sein, dass der Hub während des laufenden Betriebs (laufende Antriebseinrichtung) verstellbar ist. Hierfür kann die Hubeinstellvorrichtung am Gehäuse einen Hubeinstellmechanismus aufweisen, mit dem es ermöglicht ist, während des laufenden Betriebs des Handgeräts, wahlweise ergänzend auch, wenn die Antriebseinrichtung nicht in Betrieb ist, den Hub zu verändern. Beispielweise kann eine Drehhülse am Gehäuse gebildet sein. Mittels Drehen der Drehhülse ist eine Hubeinstellung ermöglicht. Alternativ können zur Hubeinstellung am Gehäuse ein Verschiebebauteil und / oder ein Verstellhebel vorgesehen sein.

Es kann ein Verfahren zum Betreiben des Handgeräts zum wiederholten lokalen Aufstechen einer menschlichen oder tierischen Haut vorgesehen sein, bei dem mittels der Hubeinstellvorrichtung im laufenden Betrieb, also bei laufender Antriebseinrichtung (Rotation der Antriebswelle), der Hub der Vor- und Zurückbewegung der Nadelaufnahme quer zur axialen Richtung der Antriebswelle eingestellt wird.

Mit Hilfe der Hubeinstellvorrichtung kann ein Abstand einer fußseitigen Lagerung des Kurbelzapfens zu dem Kopplungselement verstellbar sein. Mittels Verändern des Abstands zwischen der fußseitigen Lagerung des Kurbelzapfens, zum Beispiel an dem Mitnehmerbauteil, und dem Kopplungselement wird die Amplitude der auf das Koppelungselement eingeleiteten Bewegung verändert, wodurch sich der Hub ändert.

Alternativ oder ergänzend kann vorgesehen sein, dass der Winkel der Schrägstellung des Kurbelzapfens zur axialen Richtung der Antriebswelle veränderbar ist. Hierfür kann vorgesehen sein, Kurbelzapfen unterschiedlicher Schrägstellung zu verwenden. Alternativ kann ein schräggestellter Kurbelzapfen vorgesehen sein, welcher an dem den Kurbelzapfen aufnehmenden Bauteil mittels Drehen in unterschiedlichen Schrägstellungen verstellbar ist, wobei der Kurbelzapfen in den verschiedenen Drehstellungen einrasten kann.

Der Kurbelzapfen kann lösbar gelagert sein. Auf diese Weise kann in einer Ausgestaltung eine Austauschbarkeit des Kurbelzapfens erreicht werden. Beispielsweise kann so der Kurbelzapfen im Fall von Verschleiß oder Abnutzung ausgetauscht werden, wahlweise auch zusammen mit dem Mitnehmerbauteil, wobei letztere auch möglich ist, wenn Kurbelzapfen und Mitnehmerbauteil nicht lösbar miteinander verbunden sind. Es kann vorgesehen sein, dass der in dem Gehäuse angeordnete Kurbelzapfen über eine Gehäuseöffnung zugänglich ist, die mit einem abnehmbaren Gehäusedeckel verschlossen ist. Die Gehäuseöffnung kann zum Beispiel an einer Stirnseite, der Antriebseinrichtung gegenüberliegend, am Gehäuse gebildet sein. Der Gehäusedeckel kann mehrteilig ausgeführt sein, wobei ein Innendeckel des abnehmbaren Gehäusedeckels eine Aufnahme für einen Abschnitt des Kurbelzapfens aufweisen kann. Auch bei einer einteiligen Ausführung des Gehäusedeckels kann eine solche Aufnahme auf der Innenseite des Gehäusedeckels vorgesehen sein.

Eine Ausführungsform sieht vor, dass das Gehäuse mit mehreren Gehäusemodulen gebildet ist, wobei in einem Antriebsmodul die Antriebseinrichtung und in einem Stechmodul die Stecheinrichtung angeordnet ist. Die Gehäusemodule können lösbar oder nicht lösbar miteinander verbunden sein. In einer Ausgestaltung von lösbar miteinander verbundenen Gehäusemodulen kann das Stechmodul, welches auch als Nadelmodul bezeichnet werden kann, als sterilisiertes Einwegmodul ausgeführt sein.

Der Wandlungsmechanismus kann teilweise in dem Antriebsmodul und teilweise in dem Stechmodul gebildet sein. Hierbei kann vorgesehen sein, dass das Kopplungsbauteil in dem Stechmodul angeordnet ist, wohingegen wenigstens der Kurbelzapfen in dem Nadelmodul aufgenommen ist. Sind Antriebsmodul und Nadelmodul lösbar miteinander verbunden, kann zum Ausbilden des Wandlungsmechanismus der Kurbelzapfen beim Anbringen des Nadelmoduls an dem Antriebsmodul zum Beispiel in die zugeordnete Führung an dem Kopplungsbauteil eingesteckt werden.

An dem Gehäuse kann eine Schwingungsdämpfungseinrichtung gebildet sein. Hierzu können in einem Raum am Gehäuse stoßdämpfend wirkende Partikel angeordnet sein, zum Beispiel Kugelpartikel. Die Partikel können aus Wolfram bestehen. Die Partikel können miteinander starr oder flexibel verbunden sein. Es kann ein strömungsfähiges viskoses Medium oder ein viskoelastisches Medium die Partikel umgeben. Statt einzelner oder aller Partikel kann auch ein solitärer Körper vorgesehen sein. Der Raum kann mit einer verschließbaren Öffnung versehen sein.

Es kann eine zumindest in Teilen mit dem Antrieb rotierende Einrichtung vorgesehen sein, die durch eine eigene einstellbare Massenunwucht einen Schwingungsausgleich im physikalischen Gesamtsystem herbeiführt. Es kann vorgesehen sein, die Massenunwucht der Einrichtung abhängig und / oder unabhängig von der Einstellung des Hubs zu beeinflussen, so dass das Ausmaß des Schwingungsausgleichs an die physikalischen Systemforderungen bei unterschiedlicher Hubeinstellungen anpassbar ist.

In Verbindung mit dem Antriebsmodul für ein Handgerät zum wiederholten lokalen Aufstechen einer menschlichen oder tierischen Haut gelten die im Zusammenhang mit Ausführungen des Handgerätes gemachten Erläuterungen entsprechend und umgekehrt. Die in Verbindung mit dem Antriebsmodul und / oder dem Handgerät zum wiederholten lokalen Aufstechen einer menschlichen oder tierischen Haut erläuterten Ausführungen gelten entsprechend für das Verfahren zum Betreiben des Antriebsmoduls oder des Handgeräts.

### Beschreibung von Ausführungsbeispielen

Im Folgenden werden weitere Ausführungsbeispiele unter Bezugnahme auf Figuren einer Zeichnung näher erläutert. Hierbei zeigen:
- Fig. 1: eine schematische Darstellung eines Handgerätes zum wiederholten lokalen Aufstechen einer menschlichen oder tierischen Haut von der Seite,
- Fig. 2: eine schematische Darstellung des Handgerätes aus Fig. 1 von oben,
- Fig. 3: eine perspektivische Querschnittsdarstellung eines Abschnittes des Handgerätes aus Fig. 1,
- Fig. 4: eine schematische Darstellung einer Antriebseinrichtung mit Kurbelzapfen in verschiedenen Drehstellungen mit einem Hub h₁, wobei eine Kugelgelenkentkopplung vorgesehen ist,
- Fig. 5: eine schematische Darstellung der Antriebseinrichtung mit Kurbelzapfen aus Fig. 4 in verschiedenen Drehstellungen mit einem Hub h₂ < h₁,
- Fig. 6: eine schematische Darstellung einer Antriebseinrichtung mit Kurbelzapfen in verschiedenen Drehstellungen mit einem Hub h₁, wobei keine Kugelgelenkentkopplung vorgesehen ist,
- Fig. 7: eine schematische Darstellung der Antriebseinrichtung mit Kurbelzapfen aus Fig. 6 in verschiedenen Drehstellungen mit einem Hub h₂ < h₁, und
- Fig. 8: schematische Darstellungen von Kopplungsbauteilen zum Koppeln an einen schräg stehenden Kurbelzapfen.

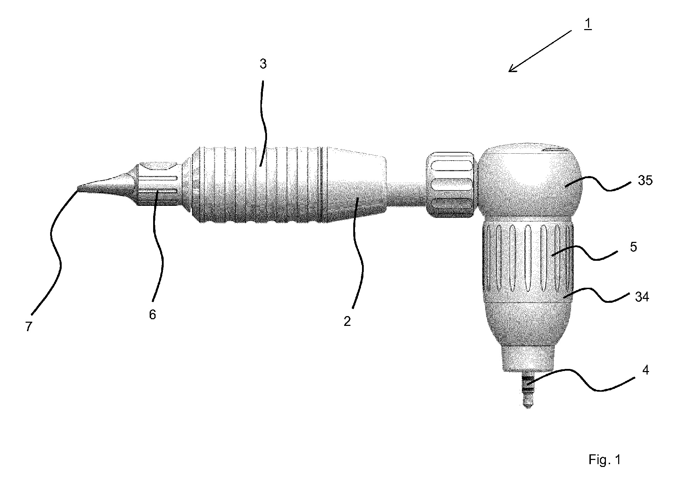

Fig. 1 zeigt eine schematische Darstellung eines Handgerätes 1 zum wiederholten lokalen Aufstechen einer menschlichen oder tierischen Haut. An einem Gehäuse 2 ist außen ein Handgriff 3 gebildet. Bei der dargestellten Ausführungsform ist in dem Gehäuse 2 ein Elektromotor als Antrieb aufgenommen, welcher über eine Steckereinrichtung 4 an eine Energieversorgung angeschlossen werden kann. Es kann vorgesehen sein, dass über die Steckereinrichtung 4 auch Signal- oder Steueranschlüsse realisiert werden, zum Beispiel zum Verbinden des Handgerätes mit einer externen Steuereinrichtung (nicht dargestellt), über die zum Beispiel eine Drehzahlregelung für den Elektromotor bereitgestellt sein kann.

Es sind ein Antriebsmodul 5 sowie ein Stech- oder Nadelmodul 6 vorgesehen. Das Stechmodul 6 kann ausgetauscht werden. Im Stechmodul 6 sind in üblicher Weise ein oder mehrere Nadeln aufgenommen, die im Betrieb durch eine vordere Gehäuseöffnung 7 aus- und eingefahren werden können. Die eine oder die mehreren Nadeln ihrerseits sind an einer Nadelaufnahme (nicht dargestellt) in üblicher Weise angeordnet.

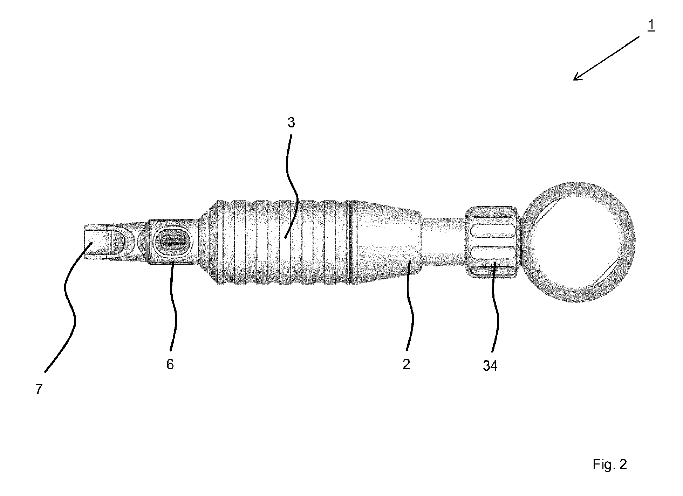

Fig. 2 zeigt das Handgerät aus Fig. 1 von oben.

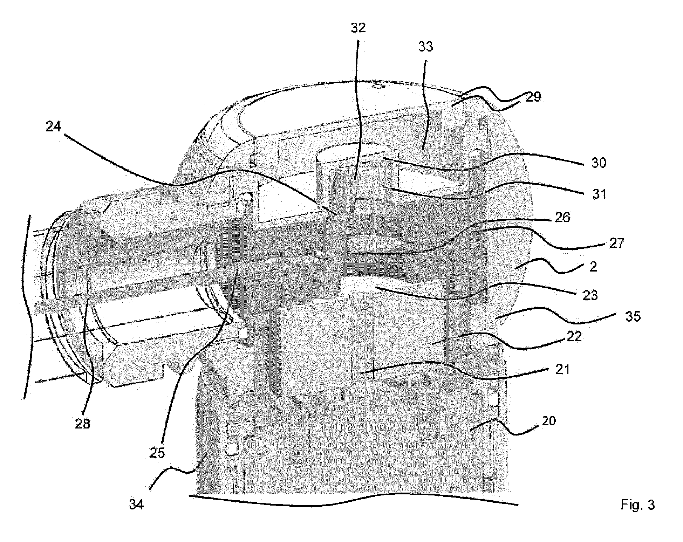

Fig. 3 zeigt eine perspektivische Querschnittsdarstellung eines Abschnittes des Handgerätes 1. Einem Elektromotor 20 gegenüberliegend ist auf einer Antriebswelle 21 ein als Mitnehmer ausgeführtes Verbindungs- oder Lagerbauteil 22 angeordnet. Auf einer Stirnseite 23 des Verbindungsbauteils 22 ist ein in der dargestellten Ausführungsform als Stift ausgeführter und schräg gestellter Kurbelzapfen 24 angeordnet, welcher seinerseits an einem verschiebbar gelagerten Kopplungsbauteil 25 in eine Ausnehmung 26 eingreift und diese in der gezeigten Betriebsstellung durchgreift.

Im Betrieb wird mit Hilfe des Elektromotors 20 eine rotierende Antriebsbewegung der Antriebswelle 21 bereitgestellt, wodurch sich das Verbindungsbauteil 22 dreht, was eine Bewegung des Kurbelzapfens 24 um die axiale Richtung der Antriebswelle 21 zur Folge hat.

Das Verbindungsbauteil 22 und der Kurbelzapfen 24 wie auch das Kopplungsbauteil 25 sind Bestandteil eines Wandlungsmechanismus, mit dem die von dem Elektromotor 20 über die Antriebswelle 21 bereitgestellte Drehbewegung in eine lineare Vor- und Zurückbewegung des Kopplungsbauteils 25 gewandelt wird. Das Kopplungsbauteil 25, bei dem die Ausnehmung 26 im proximalen Bereich angeordnet ist, bewegt sich im Betrieb in einer Führung 27, die hier flach und schlitzförmig ausgeführt ist. Alternativ zur Aufnahme des Kurbelzapfens 24 in der Ausnehmung 26 kann der Kurbelzapfen 24 über ein Kugelgelenk (nicht dargestellt) mit dem Kopplungsbauteil 25 verbunden sein. Eine solche Verbindung bewirkt ebenfalls das Vorund Zurückbewegen des Kopplungsbauteils 25 in der Führung 27, wenn sich der Kurbelzapfen 24 infolge der drehenden Antriebsbewegung im Betrieb um die axiale Richtung der Antriebswelle 21 bewegt. Es kann eine Kugelgelenk-Lagereinheit vorgesehen sein, die mehrere Funktionen wie Drehung, Gleiten, Taumeln bzw. Kreiseln an dieser Stelle realisiert.

Ein distales Ende 28 des Kopplungsbauteils 25 koppelt direkt oder indirekt an die eine oder mehrere Nadeln tragende Nadelaufnahme (nicht dargestellt), so dass diese im Betrieb repetierend vor- und zurückbewegt werden kann, insbesondere entlang einer geraden Bewegungsbahn.

Bei der dargestellten Ausführungsform in Fig. 3 ist am Gehäuse 2 ein Gehäusedeckel 29 abnehmbar angeordnet, welcher auf einer Innenseite 30 einen offenen Aufnahmeraum 31 für ein distales Ende 32 des Kurbelzapfenes aufweist. In dem Aufnahmeraum 31 bewegt sich das distale Ende 32 im Betrieb. Es kann vorgesehen sein, dass der Kurbelzapfen 24 lösbar an dem Verbindungsbauteil 22 aufgenommen ist, zum Beispiel eingesteckt oder eingeschraubt. Hierdurch ist es ermöglicht, insbesondere den Kurbelzapfen 24 auszutauschen, sei es wegen Verschleiß oder zum Einsetzen eines Kurbelzapfens mit anderer Bauform, zum Beispiel zum Bereitstellen einer geänderten Schrägstellung des Kurbelzapfens 24 zur axialen Richtung der Antriebswelle.

In einem Raum 33 im Gehäusedeckel 29 können Dämpfungsmittel angeordnet sein, zum Beispiel Partikel mit Kugelform, insbesondere Wolframpartikel, mit denen im Betrieb auftretende Schwingungen gedämpft werden (Stoßabsorption). Der Gehäusedeckel 29 kann einstückig oder, wie gezeigt, mehrstückig ausgeführt sein.

Eine Hülse 34 ist drehbar am Gehäuse 2 aufgenommen. Mittels Drehen der Hülse 34, die an ein Gewinde (nicht dargestellt) koppelt, zum Beispiel zum Bauteil 35 hin, wird die Hülse 34 in axialer Richtung der Antriebswelle 21 verlagert, insbesondere stufenlos, so dass sich mittels Relativverlagerung in axialer Richtung der Antriebswelle 21 der Abstand der Hülse 34 zur Ausnehmung 26 verändert. Hierbei werden der Elektromotor 20 sowie das hieran koppelnde Verbindungs- oder Lagerbauteil 22 mitgenommen, was schließlich die Lage der Ausnehmung 26 entlang des schrägen Kurbelzapfens 24 ändert. Im gezeigten Ausführungsbeispiel bedeutet dies, dass sich der Abstand der fußseitigen Lagerung des Kurbelzapfens 24 zur Ausnehmung 26 verändert wird. Auf diese Weise wird die Relativposition der Ausnehmung 26, wo der Abgriff der Antriebsbewegung erfolgt, entlang des schräg gestellten Kurbelzapfens 24 geändert, was quer zur axialen Richtung der Antriebswelle 21 den Abstand der Ausnehmung 26 (Abgriff) zur Antriebswelle 21 ändert und so zur Hubverstellung führt.

Das Einstellen des Hubs kann bei dieser oder anderen Ausführungen auch im laufenden Betrieb der Antriebseinrichtung ausgeführt werden, wenn sich also die Antriebswelle 21 dreht. Die beschriebene Hubeinstellung mittels Verlagern des Kopplungsorts (Abgriff oder Abgreifpunkt für Antriebsbewegung) entlang des schrägen Kurbelzapfens 24 kann auch bei Handgeräten zum Einsatz kommen, die sich hinsichtlich anderer konstruktiver Merkmale von dem gezeigten Ausführungsbeispiel unterscheiden.

So kann zum Beispiel alternativ oder ergänzend vorgesehen sein, dass das Verbindungsbauteil 22 in axialer Richtung auf der Antriebswelle 21 verlagerbar ist, wodurch zur Hubeinstellung die Relativlage des Kurbelzapfens 24 zur Ausnehmung 26 veränderbar ist. In diesem Zusammenhang kann ein am Gehäuse 2 vorgesehenes Bauteil (nicht dargestellt) eine Betätigung der Verlagerung von außen ermöglichen, wie dies oben auch für die Hülse 34 erläutert ist.

Anders ausgedrückt führt der Kurbelzapfen 24, vergleichbar einer Kegelschale, eine Rotationsbewegung um die Antriebswelle 21 aus. Eine Veränderung des Abstands zwischen Rotations- und Abtriebsebene, welche weitestgehend parallel zur Rotationsebene liegt, bewirkt einen mehr oder weniger großen Kreis vom schräg gestellten Kurbelzapfen in der Abtriebsebene um die Antriebswelle 21. Mittels besonderer Abgreifmöglichkeiten (Kopplung), zum Beispiel einem Ausgleich a) des Schrägungs- oder Anstellwinkels und b) der Kurbelzapfenrotation je Umlauf und / oder c) Realisierung der Gleitfähigkeit des Abgriffs auf dem schrägen Kurbelzapfen 24 weitestgehend senkrecht zur Rotationsebene, wird eine Hubeinstellung realisiert, die im laufenden Betrieb und auch unabhängig von der Drehzahl vorgenommen werden kann.

Es besteht hierbei die Möglichkeit, die sich auf den Nadelherausstand auswirkende verfahrensbedingte Verlagerung beider Totpunkte (Endpunkte) der abgegriffenen Linearbewegung bei der Veränderung des Hubs derart auszugleichen, dass der vordere Totpunkt (= größter Nadelherausstand) sich nicht ändert, was für den Anwender des Handgeräts sehr vorteilhaft ist.

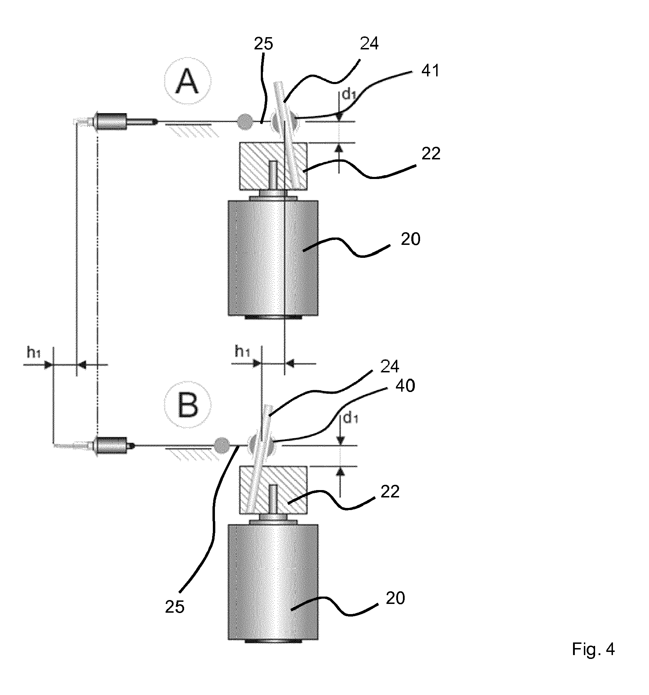

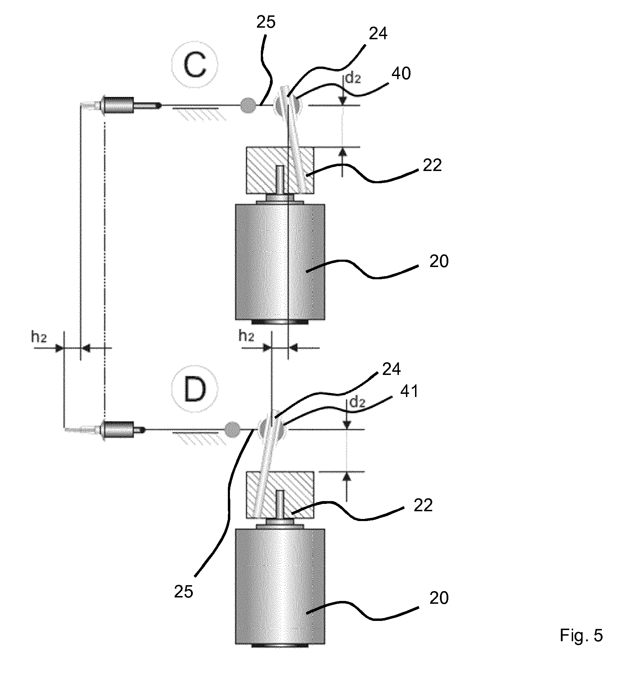

Fig. 4 eine schematische Darstellung einer Antriebseinrichtung mit Kurbelzapfen 24 in verschiedenen Drehstellungen mit einem Hub h₁, wobei im Bereich eines Abgriffs (40) eine Kugelgelenkentkopplung 41 (Kugelgelenk-Lagereinheit) vorgesehen ist Die Darstellungen A und B zeigen unterschiedliche Drehstellungen für den Kugelzapfen 24. Eine Veränderung des Abstandes d der Kopplung zwischen Kurbelzapfen 24 und Kopplungsbauteil 25 ändert den Hub h, was in Fig. 5 für einen verkleinerten Hub h₂ - bei vergrößertem Abstand d₂ > d₁ - gezeigt ist (h₂ < h₁). In Fig. 5 zeigen die beiden Darstellungen C und D wieder unterschiedliche Drehstellungen für den Kugelzapfen 24 (Endpunkte der Hubbewegung).

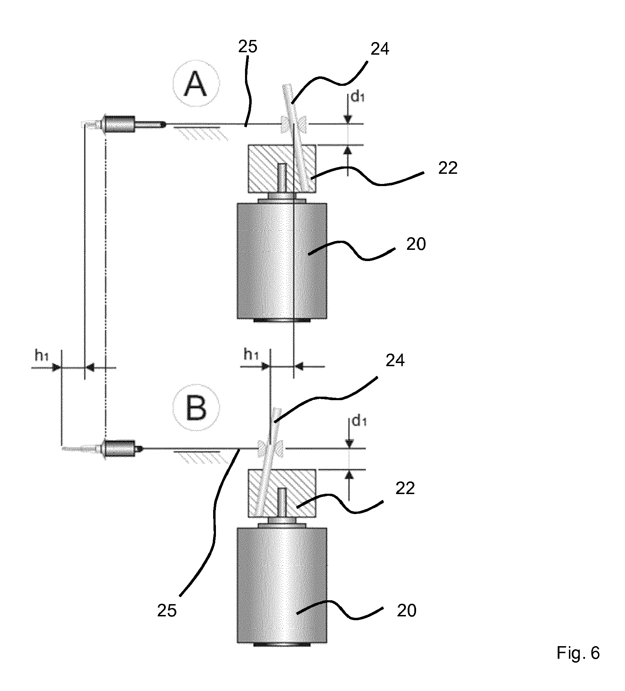

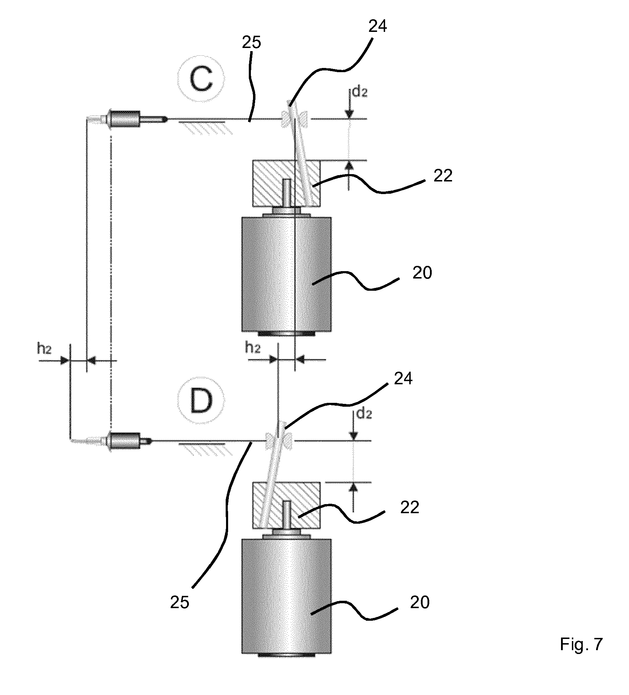

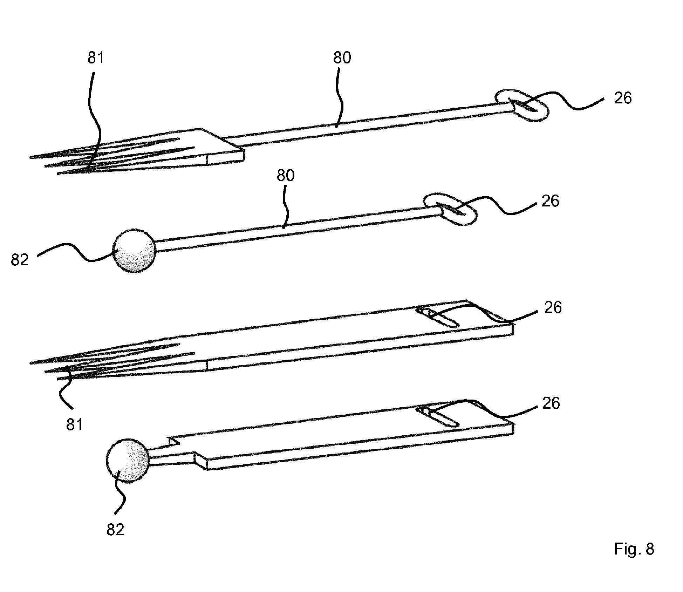

Die Fig. 6 und 7 zeigen vergleichbar den Fig. 4 und 5 unterschiedliche Drehstellungen für den Kugelzapfen 24, welcher an das als ein Flachpleuel ausgeführte Kopplungsbauteil 25 koppelt. Fig. 8 zeigt verschiedene Darstellungen von Kopplungsbauteilen zur Ankopplung an den Kurbelzapfen 24. Bei den oberen beiden Ausgestaltungen ist die Ausnehmung 26 in einem Drahtbügel 80 gebildet, wohingegen bei den unteren Ausgestaltungen in Fig. 8 ein Schlitz als Ausnehmung 26 vorgesehen ist. Zwei der Ausgestaltungen in Fig. 8 sehen eine Stecheinrichtung 81 vor, wohingegen bei den beiden anderen Ausgestaltungen ein Kopplungselement 82 gebildet ist.

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

## Patentansprüche

1. Antriebsmodul für ein Handgerät zum wiederholten lokalen Aufstechen einer menschlichen oder tierischen Haut, mit:
- einem Modulgehäuse, an dem ein Handgriff gebildet ist,
- einer Antriebseinrichtung, die in dem Modulgehäuse angeordnet ist und mit der über eine Antriebswelle (21) eine rotierende Antriebsbewegung bereitgestellt wird,
- einem an die Antriebswelle (21) koppelnden Wandlungsmechanismus, der in dem Modulgehäuse angeordnet und eingerichtet ist, die rotierende Antriebsbewegung in eine nicht rotierende Antriebsbewegung zu wandeln, und
- einer Kopplungseinrichtung, die an und / oder in dem Modulgehäuse gebildet und eingerichtet ist, an ein Stechmodul mit einer oder mehreren Stechnadeln, welche an einer Nadelaufnahme angeordnet sind, lösbar zu koppeln,
wobei der Wandlungsmechanismus einen mit der Antriebswelle (21) funktionell verbundenes und zur axialen Richtung der Antriebswelle (21) schräg gestellten Kurbelzapfen (24) aufweist, welcher infolge der rotierenden Antriebsbewegung in eine Bewegung um eine zur axialen Richtung der Antriebswelle (21) parallele Achse versetzt wird, und wobei der Kurbelzapfen (24) im Bereich eines Abgriffs (40) an ein quer zur axialen Richtung der Antriebswelle (21) hin und her verlagerbares und mit der Nadelaufnahme des Stechmoduls koppelbares Kopplungsbauteil (25) koppelt, derart, dass im Betrieb die Bewegung des Kurbelzapfens (24) in eine Vor- und Zurückbewegung des Kopplungsbauteils (25) quer zur axialen Richtung der Antriebswelle (21) gewandelt wird, **gekennzeichnet durch** eine Hubeinstellvorrichtung, mit der ein Hub der Vor- und Zurückbewegung der Nadelaufnahme quer zur axialen Richtung der Antriebswelle (21) einstellbar ist.

2. Antriebsmodul nach Anspruch 1, **dadurch gekennzeichnet, dass** der Kurbelzapfen (24) an einem Mitnehmerbauteil (22) aufgenommen ist, welches an die Antriebswelle (21) koppelt.

3. Antriebsmodul nach Anspruch 1 oder 2, **dadurch gekennzeichnet, dass** der Kurbelzapfen (24) über ein Kugelgelenk an das Kopplungsbauteil (25) koppelt.

4. Antriebsmodul nach Anspruch 1 oder 2, **dadurch gekennzeichnet, dass** der Kurbelzapfen (24) in einem Durchbruch an dem Kopplungsbauteil (25) freilaufend lagert.

5. Antriebsmodul nach mindestens einem der vorangehenden Ansprüche, **dadurch gekennzeichnet, dass** das Kopplungsbauteil (25) in einer quer zur axialen Richtung der Antriebswelle (21) ausgerichteten Führung angeordnet ist.

6. Antriebsmodul nach mindestens einem der vorangehenden Ansprüche, dadurch g e - kennzeichnet, dass mittels der Hubeinstellvorrichtung der Abgriff (40) entlang des Kurbelzapfens (24) verlagerbar ist.

7. Antriebsmodul nach mindestens einem der vorangehenden Ansprüche, **dadurch gekennzeichnet, dass** der Hub mit Hilfe der Hubeinstellvorrichtung stufenlos einstellbar ist.

8. Antriebsmodul nach mindestens einem der vorangehenden Ansprüche, **dadurch gekennzeichnet, dass** der Hub mit Hilfe der Hubeinstellvorrichtung im laufenden Betrieb der Antriebseinrichtung veränderbar ist.

9. Antriebsmodul nach mindestens einem der vorangehenden Ansprüche, **dadurch gekennzeichnet, dass** mit Hilfe der Hubeinstellvorrichtung ein Abstand einer fußseitigen Lagerung des Kurbelzapfens (24) zu dem Kopplungsbauteil (25) verstellbar ist.

10. Antriebsmodul nach mindestens einem der vorangehenden Ansprüche, **dadurch gekennzeichnet, dass** das Kurbelzapfen (24) lösbar gelagert ist.

11. Handgerät nach mindestens einem der vorangehenden Ansprüche, **dadurch gekennzeichnet, dass** an dem Gehäuse (2) eine Schwingungsdämpfungseinrichtung gebildet ist.

12. Handgerät zum wiederholten lokalen Aufstechen einer menschlichen oder tierischen Haut, mit einem Antriebsmodul nach mindestens einem der vorangehenden Ansprüche und einem hieran lösbar oder nicht lösbar angeordneten Stechmodul, wobei:
- an einem Gehäuse ein Handgriff gebildet ist,
- eine Antriebseinrichtung in dem Gehäuse (2) angeordnet ist, und mit der Antriebseinrichtung über eine Antriebswelle (21) eine rotierende Antriebsbewegung bereitgestellt wird,
- ein an die Antriebswelle (21) koppelnden Wandlungsmechanismus in dem Gehäuse (2) angeordnet und eingerichtet ist, die rotierende Antriebsbewegung in eine nicht rotierende Antriebsbewegung zu wandeln, und
- eine im Stechmodul gebildete Stecheinrichtung in dem Gehäuse (2) angeordnet ist und eine oder mehrere Stechnadeln aufweist, welche an einer Nadelaufnahme angeordnet sind, die mit dem Wandlungsmechanismus in Verbindung steht und die mit der einen oder den mehreren Stechnadeln entlang einer Bewegungsbahn wiederholt vor und zurück bewegbar ist,
- der Wandlungsmechanismus einen mit der Antriebswelle (21) funktionell verbundenen Kurbelzapfen (24) aufweist, welcher zur axialen Richtung der Antriebswelle (21) schräg gestellt ist und infolge der rotierenden Antriebsbewegung in eine Bewegung um die axiale Richtung der Antriebswelle (21) versetzt wird, und
- der Kurbelzapfen (24) im Bereich eines Abgriffs (40) an ein quer zur axialen Richtung der Antriebswelle (21) hin und her verlagerbares und mit der Nadelaufnahme in Verbindung stehendes Kopplungsbauteil (25) koppelt, derart, dass die Bewegung des Kurbelzapfens (24) in eine Vor- und Zurückbewegung des Kopplungsbauteils (25) und somit der Nadelaufnahme quer zur axialen Richtung der Antriebswelle (21) gewandelt wird,
**gekennzeichnet durch** eine Hubeinstellvorrichtung, mit der ein Hub der Vor- und Zurückbewegung der Nadelaufnahme quer zur axialen Richtung der Antriebswelle (21) einstellbar ist.

13. Handgerät nach Anspruch 12, **dadurch gekennzeichnet, dass** der Wandlungsmechanismus teilweise in dem Antriebsmodul und teilweise in dem Stechmodul gebildet ist.

14. Verfahren zum Betreiben eines Handgeräts zum wiederholten lokalen Aufstechen einer menschlichen oder tierischen Haut, bei dem
- eine Antriebseinrichtung, die in einem Gehäuse (2) angeordnet ist, über eine Antriebswelle (21) eine rotierende Antriebsbewegung bereitstellt,
- eine an die Antriebswelle (21) koppelnder Wandlungsmechanismus, der in dem Gehäuse (2) angeordnet ist, die rotierende Antriebsbewegung in eine nicht rotierende Antriebsbewegung wandelt, und
- eine Stecheinrichtung, die in dem Gehäuse (2) angeordnet ist und eine oder mehrere Stechnadeln aufweist, welche an einer Nadelaufnahme angeordnet sind, mit dem Wandlungsmechanismus in Verbindung steht und mit der einen oder den mehreren Stechnadeln entlang einer Bewegungsbahn wiederholt vor und zurück bewegt wird, wobei mittels einer Hubeinstellvorrichtung im laufenden Betrieb der Antriebseinrichtung ein Hub der Vor- und Zurückbewegung der Nadelaufnahme quer zur axialen Richtung der Antriebswelle (21) eingestellt wird.
